# EUROPÄISCHE PATENTSCHRIFT

(11) **EP 1 026 506 B1**
(45) Veröffentlichungstag und Bekanntmachung des Hinweises auf die Patenterteilung: **14.05.2003**
(21) Anmeldenummer: 00101449.7
(22) Anmeldetag: 25.01.2000
(51) Int. Cl.: G01N 33/74

(54) **Gebrauchsfertige Kalibratoren für die Bestimmung von Procalcitonin**
Ready-for-use calibrators for assaying procalcitonin
Calibrateurs prêts à l'emploi pour la détermination de la procalcitonine

(30) Priorität: 28.01.1999 DE 19903336
(43) Veröffentlichungstag der Anmeldung: 09.08.2000
(73) Patentinhaber: B.R.A.H.M.S Aktiengesellschaft, 16761 Hennigsdorf (DE)
(72) Erfinder: Bergmann, Andreas, Dr., 12351 Berlin (DE); Weglöhner, Wolfgang, Dr., 12099 Berlin (DE)
(74) Vertreter: Andrae, Steffen, Dr.

(56) Entgegenhaltungen:
- WO-A-94/04927
- DE-C- 19 600 875
- US-A- 5 385 825
- US-A- 5 639 617
- HASTEWELL, J. ET AL: "The colonic absorption of human calcitonin: The effects of increasing local concentration and co-administration with a protease inhibitor." INTERNATIONAL JOURNAL OF PHARMACEUTICS (AMSTERDAM), (1995) VOL. 126, NO. 1-2, PP. 245-251. , XP000901753
- RAITHEL, E. (1) ET AL: "LIAISON(R) PCT: An automated chemiluminescent immunoassay for the determination of procalcitonin." CLINICAL CHEMISTRY AND LABORATORY MEDICINE, (JUNE, 1999) VOL. 37, NO. SPEC. SUPPL., PP. S492. MEETING INFO.: IFC-WORLDLAB, INTERNATIONAL FEDERATION OF CLINICAL AND LABORATORY MEDICINE (17TH INTERNATIONAL AND 13TH EUROPEAN CONGRESS OF CLINICAL CHEMIST, XP000901752

## Beschreibung

Die vorliegende Erfindung betrifft gebrauchsfertige Kalibratoren für die Bestimmung von Procalcitonin in humanem Serum und Plasma, insbesondere für die Bestimmung von Procalcitonin unter Verwendung selektiver Antikörper nach immundiagnostischen Bestimmungsverfahren mit Hilfe moderner Immunoassay-Vollautomaten.

Aus den Patenten DE 42 27 454 bzw. EP 0 656 121 B1 bzw. US 5,639,617 sowie DE 196 00 875 ist es bekannt, daß die Bestimmung des Prohormons Procalcitonin bzw. von sich davon ableitenden Teilpeptiden in einem Serum oder Plasma eines Patienten, bei dem ein Sepsisrisiko besteht bzw. bei dem sepsistypische Krankheitserscheinungen festgestellt werden, ein wertvolles diagnostisches Hilfsmittel zur Früherkennung, d.h. zur Erkennung von Infektionen, die zu Sepsis führen können, zur Erkennung des Schweregrads und zur therapiebegleitenden Verlaufsbeurteilung von Sepsis und sepsisähnlichen systemischen Infektionen darstellt. Ferner ermöglicht die Bestimmung von Procalcitonin in einem Serum oder Plasma eines Patienten generell eine Abgrenzung von infektiösen lokalen und systemischen Entzündungen von entzündlichen Erkrankungen mit nicht-infektiösen Ätiologien, z.B. von Krankheitserscheinungen, die auf nicht-infektiöse Entzündungen einzelner Organe, auf postoperative Abstoßungsreaktionen oder Krebserkrankungen zurückzuführen sind. Bei lokalen Infektionen kann der Nachweis von Procalcitonin in einer Patientenprobe bei sehr viel niedrigeren Konzentrationen diagnostisch signifikant sein als im Falle von systemischen, septischen Infektionen.

Bezüglich eines Überblicks über den jüngeren Erkenntnisstand wird verwiesen auf W. Karzai et al. in Infection, Vol. 25 (1997), 6, S. 329-334 und die darin zitierte bzw. erwähnte weitere Fachliteratur.

In der älteren, nicht vorveröffentlichten PCT-Anmeldung WO 00/22439 (DE 198 47 690.6) wird ferner beschrieben, daß das mit Hilfe der bekannten Procalcitonin-Assays bei Sepsis bzw. infektiösen entzündlichen Erkrankungen im Serum oder Plasma von Patienten bestimmte endogene "Procalcitonin" nicht das vollständige Procalcitonin 1-116 (PCT116) ist, sondern ein verkürztes Procalcitonin 3-116 (PCT114), das sich allerdings in den bekannten Assays wie das vollständige Procalcitonin 1-116 verhält.

Procalcitonin kann, als ein im Patientenblut auftretendes peptidisches Biomolekül, grundsätzlich nach irgendeinem der Verfahrensprinzipien bestimmt werden, die dem Fachmann heute für die Bestimmung von Peptiden in biologischen Flüssigkeiten zur Verfügung stehen. Auch wenn die Erfindung in der vorliegenden Beschreibung anhand eines bestimmten Bestimmungsverfahrens näher erläutert wird, ist sie, wie der Fachmann sofort erkennen kann, grundsätzlich im Kontext beliebiger Bestimmungsverfahren für Procalcitonin nutzbar.

Bisher steht für die klinische Routinebestimmung von Procalcitonin (PCT) nur ein einziger kommerziell eingeführter Assay zur Verfügung, nämlich der Assay LUMItest® PCT der Anmelderin. Dieser Assay ist ein nach dem Sandwichprinzip arbeitender immunoluminometrischer Assay (ILMA) zur Bestimmung von Procalcitonin (PCT) in Humanserum und Plasma. Der Assay arbeitet nach dem grundsätzlich z.B. in dem Patent DE 42 27 454 beschriebenen Prinzip. Es werden zwei spezifische monoklonale Antikörper verwendet, von denen einer an eine Aminosäuresequenz des Procalcitonins bindet, die bei der proteolytischen Freisetzung von Calcitonin Teil des Peptids Katacalcin wird, während der andere an eine Aminosäuresequenz bindet, die Teil des reifen Calcitonins wird. Einer der Antikörper wird als Beschichtung von Coated Tubes in immobilisierter Form eingesetzt, während der andere ein Chemilumineszenzlabel aufweist. Die spezifische Bindung des markierten monoklonalen Antikörpers an die feste Phase ist nur über ein solches Peptid möglich, das sowohl die o.g. Katacalcinsequenz als auch die o.g. Calcitoninsequenz in einem Molekül vereinigt. Bezogen auf die Sequenz des vollständigen Peptids Procalcitonin binden die beiden in dem genannten Verfahren verwendeten monoklonalen Antikörper an Aminosäuresequenzen, die im Bereich der Aminosäuren 70 bis 116 des Procalcitonins zu finden sind.

Zur Messung der Konzentrationen von Biomolekülen in biologischen Flüssigkeiten wie Serum oder Plasma ist es erforderlich, daß mit Hilfe eines Tracermoleküls gewonnene Meßsignale mit den tatsächlich in der Probe vorhandenen Konzentrationen des zu bestimmenden Biomoleküls korreliert werden können. Zu diesem Zweck werden Kalibratorenlösungen verwendet, die bekannte Mengen des zu messenden Biomoleküls enthalten und die daher bei ihrer Messung mit dem zur Bestimmung eingesetzten Immunoassay Meßwerte liefern, die direkt mit bekannten Konzentrationen korreliert werden können. Indem man eine Reihe von Standards unterschiedlicher Konzentration, z.B. sechs verschiedene Standards, vermißt, wird eine Standardkurve erstellt, die dann dazu verwendet werden kann, die bei der Messung von Patientenproben erhaltenen Meßergebnisse in Konzentrationen des zu bestimmenden Biomoleküls umzurechnen.

Üblicherweise enthält daher jeder Kit zur Durchführung eines immundiagnostischen Bestimmungsverfahrens eine Reihe von "Standards". In der Regel liegen diese in Teströhrchen in lyophilisierter Form vor und werden vor dem Beginn einer Meßreihe zur Bereitung der Kalibratorlösungen mit dem sogenannten "Nullserum" versetzt, das einen weiteren Bestandteil des Kits darstellt und gleichzeitig zur Bestimmung des Blindwerts (der "Kontrolle") des Bestimmungsverfahrens dient.

Um den Aufwand für die Erstellung einer kompletten Standardkurve zu vermindern, wird insbesondere bei modernen Immunoassay-Vollautomaten meist so vorgegangen, daß man nur mit zwei Kalibratoren arbeitet, und daß man die bei der Messung der beiden Kalibratoren erhaltenen beiden Meßpunkte dazu verwendet, mit Hilfe eines Rechenprogramms eine sogenannte Masterkurve zu erstellen, die ähnlich wie die vollständige Standardkurve zur Auswertung der Meßsignale herangezogen wird. Im Vergleich mit der Erstellung einer vollständigen Standardkurve ist die mathematische Erstellung einer Masterkurve unter Verwendung von nur zwei Meßpunkten einfacher. Die mit Hilfe einer Masterkurve ermittelten Konzentrationen können jedoch von denjenigen abweichen, die mit einer vollständigen Standardkurve erhalten werden, und bei einer Auswertung von Messungen mit Hilfe von Masterkurven ist es besonders wichtig, daß die Assaybedingungen (Inkubationstemperatur, Inkubationszeit) über den ganzen Meßzeitraum konstant gehalten werden.

Im Falle der Bestimmung von Procalcitonin besteht die Schwierigkeit, daß man als Peptid für die Kalibratoren (Standards) kein vollständiges Procalcitonin-Peptid einsetzte, sondern stattdessen mit einem relativ kurzen, einfacher zu synthetisierenden Procalcitonin-Teilpeptid (PTN47) arbeitete, das nur die 47 Aminosäuren der Positionen 70 bis 116 des vollständigen Procalcitonins umfaßte. Dieses Teilpeptid weist beide Bindungsstellen für die verwendeten monoklonalen Antikörper auf, so daß es grundsätzlich für die erforderliche Eichung (Erstellung von Standardkurven bzw. Masterkurven) verwendet werden kann, wenn es einmal gegen vollständiges Procalcitonin geeicht worden war.

Es hat sich jedoch gezeigt, daß das genannte Teilpeptid insbesondere im Hinblick auf die Reaktionskinetik (Abhängigkeit der Reaktionsgeschwindigkeit von unterschiedlichen Inkubationstemperaturen) sowie andere Reaktionsbindungen ein etwas anderes Bindungsverhalten gegenüber den verwendeten Antikörpern aufwies als das in der Patientenprobe zu bestimmende native Procalcitonin. Eine zuverlässige Kalibrierung mit Hilfe relativ kurzer synthetischer Kalibratorpeptide ist. somit nur eingeschränkt und unter streng kontrollierten Bedingungen möglich. Insbesondere dann, wenn die Bestimmung mit extrem kurzen Inkubationszeiten durchgeführt werden soll, was bei modernen Immunoassay-Vollautomaten angestrebt wird, wird ein unterschiedliches Bindungsverhalten von PCT-Kalibrator-Teilpeptiden und vollständigem aktiven Procalcitonin festgestellt.

Eine weitere Schwierigkeit ergibt sich daraus, daß Procalcitonin und seine Teilpeptide in biologischen Flüssigkeiten, wie sie für die Bereitung und Messung von Standardproben (Kalibratorlösungen) verwendet werden müssen, nicht stabil sind. "Nicht stabil" bedeutet dabei, daß sich die meßbare Immunreaktivität in den Kalibratorlösungen mit der Zeit verändert. Das hat zur Folge, daß man den Assay-Kits bisher keine flüssigen Kalibratorlösungen (Standardproben) beigeben konnte, sondern daß man die Standards in lyophilisierter Form vertreiben muß und einmal hergestellte flüssige Standardproben bzw. Kalibratorlösungen nicht über längere Zeit bzw. nur im tiefgefrorenen Zustand aufbewahren konnte.

Getrocknete oder gefrorene Kalibratoren können zwar Kits für die manuelle Assaydurchführung beigegeben werden, da das technische Personal die Arbeit mit derartigen Proben gewohnt ist. Auf automatisierten Systemen, z.B. Immunoassay-Vollautomaten, sind nur getrocknet oder gefroren zur Verfügung stehende, in Lösung instabile Kalibratoren jedoch nicht einsetzbar. Derartige Vollautomaten benötigen für die Eichung der Meßergebnisse, die in der Regel mit den o.g. Masterkurven erfolgt, gebrauchsfertige, hinreichend stabile Kalibratorlösungen, in denen ein Peptid enthalten ist, das in seinem Bindungsverhalten (in seiner Bindungskinetik) dem zu bestimmenden endogenen Peptid möglichst genau entspricht.

Die Tatsache, daß für die Bestimmung von Procalcitonin bisher derartige stabile Kalibratorlösungen nicht zur Verfügung gestellt werden konnten, stellt ein prinzipielles Hindernis für die automatischen Abarbeitung von Patientenproben zur Procalcitoninbestimmung dar.

Es ist daher Aufgabe der vorliegenden Erfindung, gebrauchsfertige oder wenigstens hinreichend stabile und in ihren Bindungseigenschaften dem endogenen Procalcitonin vergleichbare Kalibratoren in Form direkt einsetzbarer oder gut lagerbarer Lösungen zur Verfügung zu stellen.

Diese Aufgabe wurde durch Kalibratorlösungen gemäß Patentanspruch 1 gelöst. Vorteilhafte Ausgestaltungen sind den Unteransprüchen zu entnehmen.

Gemäß der vorliegenden Erfindung stellt man als Kalibratorlösung eine Lösung eines Peptids mit Procalcitonin-Immunreaktivität in einem menschlichen oder tierischen Serum her, vorzugsweise in Humanserum, das einen Zusatz einer Protease-Inhibitor-Kombination enthält, die den Immunreaktivitätsverlust des Peptids unter Normalbedingungen (Raumtemperatur) verhindert oder wenigstens stark verzögert. Das ermöglicht es, die Kalibratorlösung im gebrauchsfertigen flüssigen Zustand ohne Tiefkühlung beträchtliche Zeiten zu lagern sowie ggf. sogar in diesem flüssigen Zustand herzustellen und zu vertreiben.

Das auch als "Kalibratorpeptid" bezeichnete Peptid mit Procalcitonin-Immunreaktivität kann ein Procalcitonin-Teilpeptid, z.B. das gegenwärtig in Kalibratoren eingesetzte Peptid (PTN47) sein, ist jedoch vorzugsweise ein längeres rekombinantes Procalcitonin mit einer Bindungskinetik gegenüber den im jeweiligen Immunoassay eingesetzten Antikörpern, die der des vollständigen endogenenen Procalcitonins entspricht. Vorzugsweise weist das rekombinante Procalcitonin mehr als 100 Aminosäuren des Procalcitonins auf, und besonders bevorzugt ist das rekombinante Procalcitonin eines der Peptide Procalcitonin 1-116 (PCT116), 2-116 (PCT115) oder 3-116 (PCT114), deren Herstellung nachfolgend beschrieben wird.

Durch Verwendung eines der o.g., im wesentlichen vollständigen rekombinanten Procalcitonin-Peptide werden die bei der Kalibrierung unter Verwendung von kurzen Procalcitonin-Teilpeptiden beobachteten Abhängigkeiten der Eichung von der Meßtemperatur und den Meßzeiten weitgehend ausgeräumt.

Als Proteaseinhibitoren zur Stabilisierung von Procalcitonin in einem Serum zur Herstellung von erfindungsgemäßen Kalibratorlösungen ist z.B. eine Proteaseinhibitorkombination wirksam, wie sie in den deutschen Patenten DE 38 33 936 oder DE 41 32 587 bzw. den entsprechenden europäischen Patenten EP 362773 und EP 559853 der Anmelderin beschrieben ist und die als wirksame Bestandteile nebeneinander Leupeptin, Amastatin sowie EDTA (Ethylendiamintetraessigsäure) enthält, und zwar normalerweise in Mengen von mehr als 50 µM Leupeptin, 25µM Amastatin und 4mM EDTA.

Gleichfalls wirksam haben sich im Handel erhältliche Proteaseinhibitor-Produkte erweisen, z.B. der Proteaseinhibitor-Mix, der von der Firma Boehringer Mannheim GmbH unter der Handelsbezeichnung Complete™ vertrieben wird. Unter Verwendung beider Proteaseinhibitor-Kombinationen konnten Kalibratorlösungen von vollständigen oder nahezu vollständigen rekombinanten Procalcitoninpeptiden in Humanserum hergestellt werden, in denen auch noch nach 14 Tagen Lagerung bei Raumtemperatur (22°C) keine Veränderung der Procalcitonin-Immunreaktivität feststellbar war.

Dieses Ergebnis ist auch insofern bemerkenswert, als Versuche, den Procalcitoninabbau durch Verwendung wärmedesaktivierter Humanseren oder durch Verwendung proteasefreier Trägerlösungen (PBS mit 1% BSA, proteasefrei) zu verhindern, erfolglos waren.

Nachfolgend wird die Erfindung anhand von Versuchsergebnissen näher erläutert.

In den Figuren zeigt:

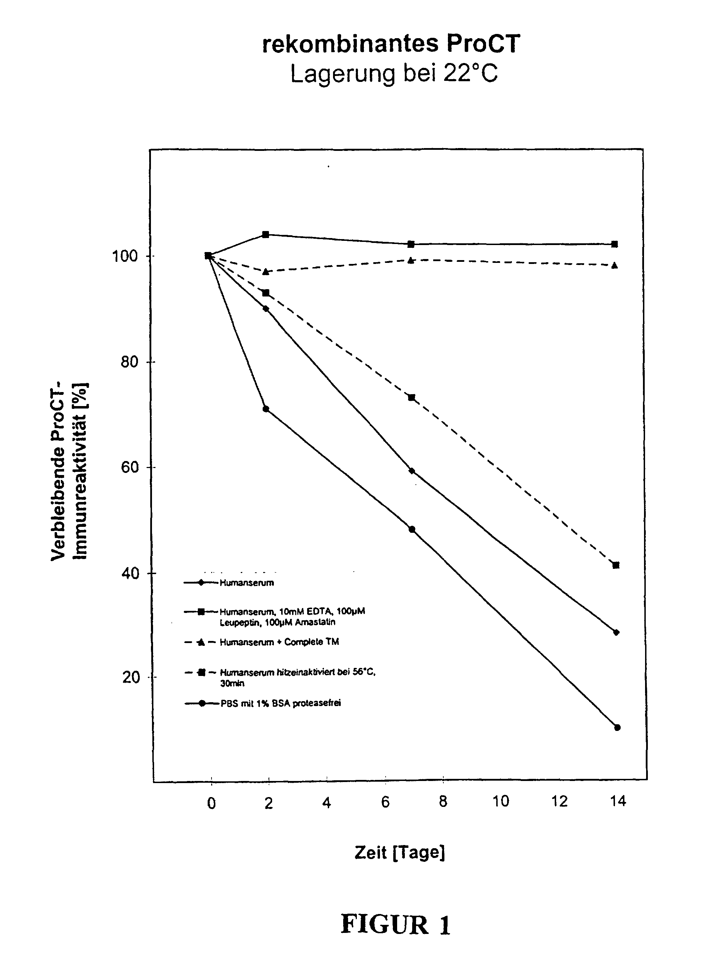

Figur 1 ein Diagramm, das die Veränderung der Anfangs-Immunreaktivität von rekombinantem PCT115 bei einer Lagerung bei 22°C in verschiedenen flüssigen Trägern innnerhalb eines Zeitraums von bis zu 14 Tagen zeigt.

### 1. Klonierung, Expression und Aufreinigung von rekombinan tem Procalcitonin

Die für Procalcitonin 1-116, 2-116 oder 3-116 (nachfolgend abgekürzt PCT116, PCT115 oder PCT114) codierenden DNA-Fragmente wurden unter Anwendung einer PCR-Amplifikation mit Hilfe geeigneter Oligonukleotidprimer aus einem humanen Schilddrüsen-cDNA-Pool isoliert. Das gewünschte Fragmente wurde mittels gängiger Methoden (Ausubel FM, Brent R, Kingston RE, Moore DD, Seidman JG, Smith JA, und Struhl K (1991), Current Protocols in Molecular Biology, John Wiley & Sons, New York) kloniert, und die korrekte Nukleotidsequenz wurde durch DNA-Sequenzierung und Vergleich mit der bekannten, für Procalcitonin codierenden DNA-Sequenz verifiziert.

Für die Expression z.B. der für PCT 115 codierenden cDNA wurde ein Vektor verwendet, der neben einem T7-Promotor eine Region enthält, die für das Signalpeptid des sog. pelB-Proteins codiert. Dieses pelB-Signalpeptid sorgt dafür, daß ein nach Klonierung und Expression entstandenes Fusionsprotein durch die cytoplasmatische Membran der für die Expression verwendeten Wirtszellen in den periplasmatischen Raum transportiert wird. Bei diesem Transportvorgang wird gleichzeitig das N-terminale Signalpeptid durch eine membranständige Signalpeptidase abgetrennt (Stader JA und Silhavy TJ (1990), Engineering Escherichia coli to secrete heterologous gene products, Methods Enzymol. 185, 166-187). Diese Vorgehensweise garantiert, daß das gefundene Expressionsprodukt exakt die gewünschte Sequenz aufweist. Bei dieser Vorgehensweise fehlt das bei anderen Methoden zur Expression notwendige N-terminale Methionin.

Nach der Klonierung der cDNA für PCT 115 in einen Vektor der genannten Art und der Transformierung von E. coli mit diesem Vektor wurde Procalcitonin 2-116 exprimiert. Die periplasmatische Fraktion mit dem exprimierten Procalcitonin 2-116 wurde auf an sich bekannte Weise isoliert (Neu HC und Heppel LA (1965), The release of enzymes from Escherichia coli by osmotic shock and during the formation of spheroblasts, J. Biol. Chem. 240, 3685-3692). Nach einer Zentrifugierung (100.000 g, 30 min, 4°C) und Filtration des flüssigen Überstands (0,2 µm) wurde das erhaltene Filtrat durch Anionen-Austauschchromatographie aufgetrennt. Die Fraktionen mit Procalcitonin-Immunreaktivität wurden vereinigt und über Umkehrphasen-HPLC an einer rpC₁₈-Säule µ Bondapak 0,4 x 30 mm (Firma Waters) gereinigt. Die Durchflußgeschwindigkeit betrug 1 ml/min, Laufmittel und Elutionsbedingungen sind der folgenden Tabelle 1 zu entnehmen.

**Tabelle 1**

| Elutionsbedingungen rp-HPLC Procalcitonin | | | |
|---|---|---|---|
| Laufmittel A | 5 % Acetonitril | | |
| | 20 mM NH₄-Acetat | | |
| Laufmittel B | 90 % Acetonitril | | |
| | 20 mM NH₄-Acetat | | |
| Gradient | 0,0 min | 100 % A | 0 % B |
| | 2,5 min | 100 % A | 0 % B |
| | 5,0 min | 89 % A | 11 % B |
| | 55,0 min | 56 % A | 44 % B |
| | 60,0 min | 0 % A | 100 % B |

Der Säulenausfluß wurde kontinuierlich auf seine Absorption bei 214 nm vermessen und in Fraktionen von 0,25 ml gesammelt. Mit Hilfe eines handelsübliche Procalcitonin-Assays (LUMItest PCT, B.R.A.H.M.S Diagnostica) wurden diejenigen Fraktionen bestimmt, in denen eine PCT-Immunreaktivität nachweisbar war.

Alle Fraktionen mit Procalcitonin-Immunreaktivität wurden vereinigt und lyophilisiert. Wie eine Überprüfung des Materials mittels SDS-PAGE ergab, war das so gewonnene Material zu mindestens 95% sauber.

Das oben für die gentechnologische Herstellung von Procalcitonin 2-116 (PCT115) beschriebene Verfahren wurde in im wesentlichen identischer Form auch zur Herstellung des vollständigen Procalcitonins 1-116 und des Procalcitonins 3-116 eingesetzt.

Die Identität der exprimierten und aufgereinigten Procalcitonin-Peptide wurde durch Massenspektrometrie (MALDI-TOF) und N-terminale Aminosäuresequenzierung bestätigt.

### 2. Bestimmung der Stabilität von Kalibratorlösungen, die PCT115 als Kalibratorpeptid enthalten

Eine auf die oben beschriebene Weise hergestellte und aufgereinigte Probe von rekombinantem Procalcitonin 2-116 (PCT 115) wurde dazu verwendet, Kalibratorlösungen in verschiedene Trägerflüssigkeiten herzustellen und die Veränderung der Procalcitonin-Immunreaktivität in den Lösungen, gemessen mit dem o.g. kommerziellen LUMItest® PCT der Anmelderin, festzustellen. Insbesondere wurde Procalcitonin 2-116 in einer solchen Menge eingesetzt, daß eine Endkonzentration von 20 ng/ml, wie sie für eine Kalibratorlösung zur Erstellung einer Masterkurve für die Procalcitoninbestimmung im Rahmen der Sepsisdiagnose typisch ist, eingestellt wurde. Es wurden die folgenden Trägerflüssigkeiten untersucht:
1. Humanserum (Handelsprodukt der Fa. Scantibodies)
2. Humanserum, bei 56°C für 30 min Hitze-inaktiviert
3. Phosphatgepufferte, physiologische Kochsalzlösung (PBS) mit 1% BSA, proteasefrei (Fa. Sigma)
4. Humanserum mit 10 mM EDTA (Merck), 100µM Leupeptin (Fa. Biomol) und 100µM Amastatin (Fa. Biomol),
5. Humanserum plus im Handel erhältlicher Protease-Inhibitor-Mix (Complete ™ der Boeringer Mannheim GmbH).

Unter Verwendung des LUMItest® PCT-Assay der Anmelderin wurde die Abbnahme der PCT-Immunreaktivität in den 5 Proben über einen Zeitraum von 14 Tagen verfolgt. Die Ergebnisse sind in Figur 1 graphisch zusammengefaßt. Es ist zu erkennen, daß die PCT-Immunreaktivität des rekombinanten PCT115 in Gegenwart der beiden Proteaseninhibitor-Kombinationen auch nach 14 Tagen noch im wesentlichen unverändert war. Im Humanserum ohne Zusätze, auch in Hitze-inaktiviertem Humanserum, sowie außerdem auch in PBS, d.h. einem Trägermedium, das kein proteolytisches Enzym enthält, nahm dagegen die meßbare PCT115-Immunreaktivität innerhalb von 14 Tagen sehr stark ab.

Durch die erstmalige Bereitstellung von stabilen Kalibratorlösungen, die als Kalibratorsubstanz das vollständige oder nahezu vollständige rekombinante Procalcitonin enthalten, wurden die Voraussetzungen dafür geschaffen, zuverlässige Procalcitonin-Bestimmungen unter Verwendung bekannter Immunoassay-Vollautomaten (z.B. dem automatischen Chemilumineszenz-Immunoassay-System ACS:180™ PLUS der Firma Chiron; im LIAISON®-System von Byk-Sangtec Diagnostica GmbH & Co. KG oder dem ELECSYS®-System der Firma Boehringer Mannheim GmbH) durchzuführen.

Die erfindungsgemäßen Kalibratorlösungen können in gebrauchsfertiger Form hergestellt und vertrieben werden. Es ist jedoch auch als Ausführungsform der Erfindung anzusehen, die Kalibratorpeptide wie bisher in Form lyophilisierter Standards herzustellen und zu vertreiben und dem Kit eine stabilisierende Proteaseinhibitorkombination als weiteren Kitbestandteil oder als Zusatz zu einer der üblichen Kitkomponenten, z.B. als Zusatz zu dem Nullserum oder in lyophiliserter Form in den Standards, hinzuzufügen. Im letzteren Falle wird die erfindungsgemäße stabile Kalibratorlösung vor Ort direkt beim Endverwender hergestellt.

Die für die Kalibratorlösungen vorgesehen Procalcitonin-Konzentrationen können in Bereichen verschiedener Größenordnung liegen, und zwar in Abhängigkeit davon, ob Procalcitonin im Zusammenhang mit systemischen (septischen) Infektionen bestimmt werden soll, bei denen es in sehr hohen Konzentrationen auftritt, oder im Zusammenhang mit lokalen Entzündungen von Organen wie z.B. der Lunge oder der Magenschleimhaut, um infektiöse von nichtinfektiösen Entzündungen zu unterscheiden. Im letzteren Falle wird mit hochsensitiven Immunoassays gearbeitet, es werden sehr niedrige PCT-Konzentrationen bestimmt, und folglich sind die PCT-Konzentrationen auch in den Kalibratoren für solche Bestimmungen sehr viel niedriger.

## Patentansprüche

1. Kalibratorlösung für Verfahren zur Bestimmung von Procalcitonin oder daraus gebildeten Teilpeptiden, die nicht das reife Calcitonin sind, in Patientenproben, wobei die Kalibratorlösung eine definierte Konzentration eines Kalibratorpeptids mit Procalcitonin-Immunreaktivität sowie Serum enthält, **dadurch gekennzeichnet, daß** die Kalibratorlösung außerdem einen Zusatz einer Proteaseinhibitor-Kombination in Form einer Kombination von Leupeptin, Amastatin und EDTA oder eines handelsüblichen Proteaseinhibitor-Mix zur Peptidstabilisierung enthält, der den Verlust der Procalcitonin-Immunreaktivität des Kalibratorpeptids verhindert und es ermöglicht, die Kalibratorlösung im gebrauchsfertigen Zustand zu lagern und/oder zu vertreiben.

2. Kalibratorlösung nach Anspruch 1, **dadurch gekennzeichnet, daß** sie als Kalibratorpeptid ein durch Rekombinationstechniken erzeugtes Procalcitonin-Peptid mit Procalcitonin-Immunreaktivität enthält, das eine Sequenz von 100 oder mehr der Aminosäuren des vollständigen Procalcitonins aufweist.

3. Kalibratorlösung nach Anspruch 2, **dadurch gekennzeichnet, daß** sie als rekombinantes Procalcitonin-Peptid ein Peptid mit den Aminosäuren 1-116 (PCT116), 2-116 (PCT115) und/oder 3-116 (PCT114) enthält.

## Claims

1. Calibrator solution for methods for the determination of procalcitonin or partial peptides formed therefrom which are not the mature calcitonin in samples of patients, said calibrator solution containing a defined concentration of a calibrator peptide having procalcitonin immune reactivity and serum, **characterized in that** the calibrator solution further contains an addition of a protease inhibitor combination in the form of a combination of leupeptin, amastatin and EDTA, or a commercially available mix of protease inhibitors for peptide stabilisation, which addition prevents the loss of procalcitonin immune reactivity of said calibrator peptide and makes it possible to store and/or distribute said calibrator solution in a ready-for-use form.

2. Calibrator solution according to claim 1, **characterized in that** it contains as said calibrator peptide a recombinantly produced procalcitonin peptide having procalcitonin immune reactivity and having a sequence of 100 or more of the amino acids of the complete procalcitonin.

3. Calibrator solution according to claim 2, **characterized in that** it contains as said recombinant procalcitonin peptide a peptide having the amino acids 1-116 (PCT116), 2-116 (PCT115) and/or 3-116 (PCT114).

## Revendications

1. solution d'étalonnage pour des procédés de détermination, dans des échantillons de patients, de procalcitonine ou de peptides partiels dérivés de celle-ci et qui ne sont pas la calcitonine mature, la solution d'étalonnage contenant une concentration définie en un peptide d'étalonnage qui présente une immunoréactivité vis-à-vis de la procalcitonine, ainsi que du sérum, **caractérisée en ce que** la solution d'étalonnage contient en outre un ajout d'une combinaison d'inhibiteur de protéase qui présente la forme d'une combinaison de leupeptine, d'anastatine et d'EDTA ou un mélange, disponible sur le marché, d'inhibiteur de protéase pour stabiliser les protéines, qui inhibe la a perte d'immunoréactivité du peptide d'étalonnage vis-à-vis de la procalcitonine et permet de conserver et/ou d'amener la solution d'étalonnage dans un état actif.

2. solution d'étalonnage selon la revendication 1, **caractérisée en ce que**, comme peptide d'étalonnage, elle contient un peptide de procalcitonine produit par des techniques de recombinaison, qui présente une immunoréactivité vis-à-vis de la procalcitonine et qui présente une séquence de 100 acides aminés ou plus de la procalcitonine complète.

3. Solution d'étalonnage selon la a revendication 2, **caractérisée en ce que**, comme peptide de procalcitonine recombiné, elle contient un peptide qui présente les acides aminés 1-116 (PCT116), 2-116 (PCT115) et/ou 3-116 (PCT114).
